# EUROPEAN PATENT APPLICATION

(11) **EP 4 410 349 A1**
(43) Date of publication of application: **07.08.2024**
(21) Application number: 23154968.4
(22) Date of filing: 03.02.2023
(51) Int. Cl.: A61M 25/00

(54) **A MICROCATHETER FOR INTERVENTIONAL CARDIOVASCULAR AND/OR NEUROVASCULAR APPLICATIONS**

(71) Applicant: IMDS R&D BV, 9301 NZ Roden (NL)
(72) Inventor: ABBRING, Drewes Pieter, 9301 NZ Roden (NL); ELEVELD, Rolf, 9301 NZ Roden (NL); VAN DE WEG, Joachim, 9301 NZ Roden (NL); LAMERS, Edwin Adriaan Derk, 9301 NZ Roden (NL); SCHULTING, Edwin Alexander, 9301 NZ Roden (NL)
(74) Representative: V.O.

(57) **Abstract**

A microcatheter (1) with a distal tip (15) comprises a tubular catheter wall (2), which comprises a proximal section (6) and a distal section (14). The catheter wall comprises a skeleton, an inner liner and an outer laminate. A continuous-skeleton-range is defined as at least extending distally from a first position (21) to far into the distal tip (15). Everywhere in said continuous-skeleton-range the skeleton is continuously extending to form at least one elongated continuous reinforcement structure. Two overall skeleton cut-away ratios of all parts of the distal section and of all parts of the distal tip, respectively, within said continuous-skeleton-range are at least 50% and at least 20%, respectively. The invention provides a solution for optimizing the flexibility of the catheter when navigating deep into very fine vascular tortuous pathways, while at the same time enabling the microcatheter to penetrate and pass highly clogged stenoses.

## Description

The invention relates to a catheter, being a microcatheter for interventional cardiovascular and/or neurovascular applications, wherein the catheter comprises a tubular catheter wall, which is surrounding a lumen structure of the catheter, the catheter wall having a proximal end, a distal end and a longitudinal direction extending from the proximal end to the distal end, wherein the catheter wall has a length of at least 1000 mm from the proximal end to the distal end along the longitudinal direction.

In said cardiovascular and neurovascular applications, such a catheter must be angled through the tortuous bends and curves of a vasculature passageway to arrive at the targeted anatomy. Such a catheter requires sufficient flexibility, particularly closer to its distal end, to navigate such tortuous pathways. However, other design aspects must also be considered. For example, the catheter must also be able to provide sufficient torquability (i.e., the ability to transmit torque applied at the proximal end all the way to the distal end), pushability (i.e., the ability to transmit axial push to the distal end), and structural integrity for performing intended medical functions.

US 2019/0255290 A1 discloses in Fig. 3 an example of an interventional cardiovascular catheter specifically designed for being positioned near the aortic root. Figs. 4A-4D and Fig. 5 of US 2019/0255290 A1 show some examples of skeletons that can be applied in the various longitudinal sections of the catheter of Fig. 3 of US 2019/0255290 A1, wherein such a skeleton is an interrupted tubular reinforcement wall obtained by microfabricating a cut pattern in a fully uninterrupted tubular reinforcement wall made of a reinforcement material. The catheter of Fig. 3 of US 2019/0255290 A1 has been particularly designed for providing a relatively sharp bend 54 (see Fig. 2 of US 2019/0255290 A1) to extend across the aortic root 16 and reach into the artery 24 (see Fig. 2 of US 2019/0255290 A1). Especially the proximal-intermediate section 106 of Fig. 3 of US 2019/0255290 A1), which includes the one-beam configuration of the skeleton of Fig. 4D of US 2019/0255290 A1) has been designed for providing said relatively sharp bend 54. US 2019/0255290 A1 further dicloses that the catheter of Fig. 3 of US 2019/0255290 A1 may have an inner liner and/or an outer laminate which are extending distally beyond the skeleton to form an atraumatic distal tip 116 of the catheter.

As compared to the above-mentioned particular design object underlying the catheter of the above-mentioned document US 2019/0255290 A1, the present invention has a different object, which is summarized as follows.

It is an object of the present invention to provide a solution for optimizing the flexibility of the microcatheter when navigating deep into the very fine cardiovascular and neurovascular tortuous pathways, while at the same time enabling the microcatheter to penetrate and pass highly clogged stenoses.

For that purpose the invention provides a catheter according to the appended independent claim 1. Preferable embodiments of the invention are provided by the appended dependent claims 2-7.

Hence, the invention provides a catheter, being a microcatheter for interventional cardiovascular and/or interventional neurovascular applications,
wherein the catheter comprises a tubular catheter wall, which is surrounding a lumen structure of the catheter, the catheter wall having a proximal end, a distal end and a longitudinal direction extending from the proximal end to the distal end, wherein the catheter wall has a length of at least 1000 mm from the proximal end to the distal end along the longitudinal direction;
wherein the catheter wall comprises:
   - a proximal section, being defined as extending distally along the longitudinal direction from the proximal end to a first position at 100 mm proximal from the distal end, and
   - a distal section, being defined as extending distally along the longitudinal direction from said first position to the distal end,
wherein the distal section comprises a distal tip of the catheter, which is defined as extending proximally along the longitudinal direction from the distal end to a second position at 7 mm proximal from the distal end;
and wherein the catheter wall at least in a part of the distal section comprises:
   - a skeleton, which provides reinforcement of the catheter wall in the longitudinal direction, as well in a circumferential direction around the longitudinal direction, wherein the skeleton is an interrupted tubular reinforcement wall obtained by microfabricating a cut pattern in a fully uninterrupted tubular reinforcement wall made of a reinforcement material, and wherein the reinforcement material of the skeleton, everywhere in a continuous-skeleton-range along the longitudinal direction, is continuously extending to form at least one elongated continuous reinforcement structure all along said continuous-skeleton-range, wherein said continuous-skeleton-range is at least extending distally along the longitudinal direction from the first position to a position at 2 mm proximal from the distal end,
   - an inner liner, which at least in said continuous-skeleton-range is co-axially surrounded by said skeleton relative to a center line of the catheter wall, and
   - an outer laminate, which at least in said continuous-skeleton-range is co-axially surrounding said skeleton relative to the center line of the catheter wall;
and wherein an overall skeleton cut-away ratio of a longitudinal section of the catheter wall in a longitudinal range along the longitudinal direction is defined as the percentage of:
   - the volume of all parts, within said longitudinal range, of said reinforcement material being cut-away from said fully uninterrupted tubular reinforcement wall according to said microfabricated cut pattern,
relative to
   - the volume of all parts, within said longitudinal range, of said reinforcement material of said fully uninterrupted tubular reinforcement wall;
      and wherein the overall skeleton cut-away ratio of all parts of the distal section that are within said continuous-skeleton-range is at least 50%,
      and wherein the overall skeleton cut-away ratio of all parts of the distal tip that are within said continuous-skeleton-range is at least 20%.

In the above-recited features of the present invention, the key features of the invention are that:
i. in said continuous-skeleton-range which is at least extending distally from said first position at 100 mm proximal from the distal end to a position as far as 2 mm proximal from the distal end, the reinforcement material of the skeleton is continuously extending to form at least one elongated continuous reinforcement structure all along said continuous-skeleton-range;
ii. the overall skeleton cut-away ratio of all parts of the distal section that are within said continuous-skeleton-range is at least 50%; and
iii. the overall skeleton cut-away ratio of all parts of the distal tip that are within said continuous-skeleton-range is at least 20%.

Thanks to these combined key features (i), (ii) and (iii) the microcatheter according to the invention has a good flexibility in the distal section, including the distal tip. It is noted that distal tips of microcatheters typically have lengths between 3 and 7 mm, with exceptions up to 8 or 9 mm for long distal tips. The flexibility of, especially, the distal tip is a determinative factor to successively navigate the microcatheter through the tortuous bends and curves of a vasculature passageway to arrive at a targeted anatomy. After all, once the distal tip has passed a challenging curve, the remainder of the microcatheter generally follows. Furthermore, a distal tip of a microcatheter often has to penetrate and pass highly clogged stenoses. For that purpose the distal tip additionally needs to have good penetration behaviour and structural strength. The fact that according to key feature (i) the skeleton of a catheter according to the invention is continuously extending from said first position at 100 mm proximal from the distal end to a position as far as 2 mm proximal from the distal end, provides the distal tip with good penetration behaviour and structural strength to successfully penetrate and pass highly clogged stenoses. Key features (ii) and (iii) combined with key-feature (i) additionally provide the distal section and the distal tip with the flexibility properties to successfully navigate the microcatheter through challenging tortuous bends and curves of vasculature passageways.

Known distal tips of microcatheters, on the other hand, are either atraumatic tips having no (metal) reinforcement material at all, or distal tips having a totally uninterrupted tubular metal reinforcement wall. The known atraumatic distal tips are generally unsuitable to penetrate and pass a highly clogged stenosis of a vasculature, while the known uninterrupted metal distal tips are generally unsuitable to pass challenging tortuous bends and curves of the vasculature. Furthermore it is noted that a number one serious problem of known uninterrupted metal distal tips that have been affixed to catheters is, that such a distal tip sometimes breaks off from the catheter during an interventional procedure. The distal tip of a microcatheter according to the present invention, on the other hand, has very good structural integrity with the remainder of the distal section of the catheter, since the reinforcement material of the skeleton is continuously extending in distal direction at least from said first position at 100 mm proximal from the distal end to a position at 2 mm proximal from the distal end.

It is noted that manufacturing said very high overall skeleton cut-away ratio (at least 50%) of the distal section according to the invention requires inventive measures to solve several problems in the present case where the catheter concerned is a microcatheter for interventional cardiovascular and/or interventional neurovascular applications having said inner liner surrounded by said skeleton. The reason is that the inner liner for said cardiovascular and/or neurovascular applications typically is a tube made of, for example, PTFE (polytetrafluoroethylene, e.g. Teflon^{®}), PVDF (polyvinylidene fluoride, e.g. Kynar^{®}) or HDPE (high-density polyethylene), which during manufacturing of the microcatheter is axially inserted into the skeleton, and which is then expanded against the skeleton by inflation and heat. For said very high cut-away ratios the problems arise that the tube made of, for example, PTFE, PVDF or HDPE being inflated against the skeleton will bulge out of the large cut-outs in the skeleton material and/or will break under the inflation pressure. Another problem, especially for skeletons made of less strong material, is that the skeleton may severely deform and/or crack and/or break under the inflation pressure due to the fact that the skeleton is severely weakened by the large cut-outs in the skeleton material.

In connection therewith, the present invention inter alia is based on the new insights, as disclosed herein, that said problems can be solved by introducing, temporarily during the manufacturing of a catheter according to the invention, a heat-shrinkable outer tube over the outer side of the skeleton, so that the heat-shrinkable outer tube temporarily closes off the cut-outs in the skeleton material during said expansion of the inner liner by inflation and heat, while at the same time the heat-shrinkable outer tube temporarily reinforces the skeleton during said expansion of the inner liner by inflation and heat. After the inner liner has been successfully expanded against the skeleton and after the catheter under construction has been cooled, the temporary heat-shrinkable outer tube can be removed from the outer side of the skeleton, and be replaced by the final outer laminate over the outer side of the skeleton.

Alternatively, another new insight, as disclosed herein, is that said problems can be solved by performing the following manufacturing steps for assembling the inner liner and the outer laminate to the skeleton in case of said very high overall skeleton cut-away ratio (at least 50%) of the distal section of the catheter wall of a catheter according to the invention. Firstly, a tubular base layer of fluorinated ethylene propylene (FEP) is extruded and the inner liner is extruded over the base layer. Next, the FEP base layer together with the inner liner is axially inserted into the skeleton. Next, the outer laminate is slided over the outer side of the skeleton. Thereafter, a temporary heat-shrinkable outer FEP tube is slided over the outer laminate, and the total assembly is heated. During heating the temporary heat-shrinkable outer FEP tube provides high compression onto the assembly of the inner liner, the skeleton and the outer laminate. After the catheter under construction has been cooled, the temporary heat-shrinkable outer tube can be removed from the outer side of the skeleton, and the FEP base layer can be axially pulled out of the assembly, making use of the favourable properties of FEP with respect to being pulled. Thus, a very close fit of the inner liner against the inner side of the skeleton is obtainable.

Another advantage of the present invention is that, thanks to the very high overall skeleton cut-away ratio (at least 50%) of the distal section of the catheter wall of a catheter according to the invention, and thanks to the above-mentioned insights as disclosed herein, the invention allows for efficiently embedding a radiopaque element (e.g. a radiopaque marker) in the catheter wall, since the radiopaque element, can be perfectly located in a space of the cut pattern, said space being bounded by the skeleton, the inner layer and the outer laminate.

In a preferable embodiment of a catheter according to the invention, said continuous-skeleton-range is at least extending distally along the longitudinal direction from the first position to a position at 0.5 mm proximal from the distal end. Thus the skeleton is distally extending yet farther into the distal tip, which further promotes good penetration behaviour of the distal tip to successfully pass highly clogged stenoses.

More preferably, said continuous-skeleton-range is at least extending distally along the longitudinal direction from the first position to a position at 0.2 mm proximal from the distal end. Thus the skeleton is distally extending still yet farther into the distal tip, which yet further promotes good penetration behaviour of the distal tip to successfully pass highly clogged stenoses.

In another preferable embodiment of a catheter according to the invention, the overall skeleton cut-away ratio of said all parts of the distal section that are within said continuous-skeleton-range is at least 65%, and wherein the overall skeleton cut-away ratio of said all parts of the distal tip that are within said continuous-skeleton-range is at least 40%. This promotes to further increase flexibility in the distal section and the distal tip, which further helps to successively navigate the microcatheter through the tortuous bends and curves of a vasculature passageway to arrive at a targeted anatomy.

More preferably, the overall skeleton cut-away ratio of said all parts of the distal section that are within said continuous-skeleton-range is at least 80%, and the overall skeleton cut-away ratio of said all parts of the distal tip that are within said continuous-skeleton-range is at least 60%. This promotes to yet further increase flexibility in the distal section and the distal tip, which yet further helps to successively navigate the microcatheter through the tortuous bends and curves of a vasculature passageway to arrive at a targeted anatomy.

In another preferable embodiment of a catheter according to the invention, a radiopaque element is embedded in the catheter wall in that the radiopaque element is located in a space of the cut pattern, said space being bounded by the skeleton, the inner layer and the outer laminate.

In another preferable embodiment of a catheter according to the invention, the skeleton, distally along the longitudinal direction, has a distal skeleton end in the form of a circular edge of the skeleton, said circular edge having a center point at the center line of the catheter wall. Such a distal skeleton end in the form of a circular edge of the skeleton further promotes good penetration behaviour of the distal tip to successfully pass highly clogged stenoses, while at the same time improving the structural integrity of the skeleton, and while still allowing for good flexibility of the distal tip.

In another preferable embodiment of a catheter according to the invention, the distal tip is a tapered distal tip in that the distal tip has an outer diameter, which, distally along the longitudinal direction, is gradually narrowing up to the distal end. Such a tapered distal tip further promotes good penetration behaviour of the distal tip. The tapering of the tapered distal tip may, by way of example only, be oriented at an angle of about 5 degrees to the center line of the catheter wall. Manufacturing of the tapering of the tapered distal tip may for example be done after the inner liner and the outer laminate of the catheter wall have been assembled to the skeleton's reinforcement material of the distal tip. Alternatively, however, manufacturing of the tapering of the tapered distal tip may also be done before the inner liner and/or the outer laminate of the catheter wall have been assembled to the skeleton's reinforcement material of the distal tip.

In the following, the invention is further elucidated with reference to a non-limiting embodiment and with reference to the schematic figures in the appended drawing, in which the following is shown.
Fig. 1 shows, in a longitudinal side view, an example of an embodiment of a catheter according to the invention, wherein in Fig. 1 the proximal section and the distal section of the catheter wall, as well as the distal tip of the catheter have been indicated by brace marks.
Fig. 2A shows, within the above-mentioned continuous-skeleton-range, a short longitudinal section of the catheter wall of the catheter of Fig. 1, wherein the shown longitudinal section comprises a part of the skeleton of the catheter wall, and wherein the shown view is a longitudinal sectional view, which contains the center line of the catheter wall.
Fig. 2B shows a cross-section through the longitudinal section of the catheter wall of Fig. 2A, wherein the shown cross-section is perpendicular to the center line.

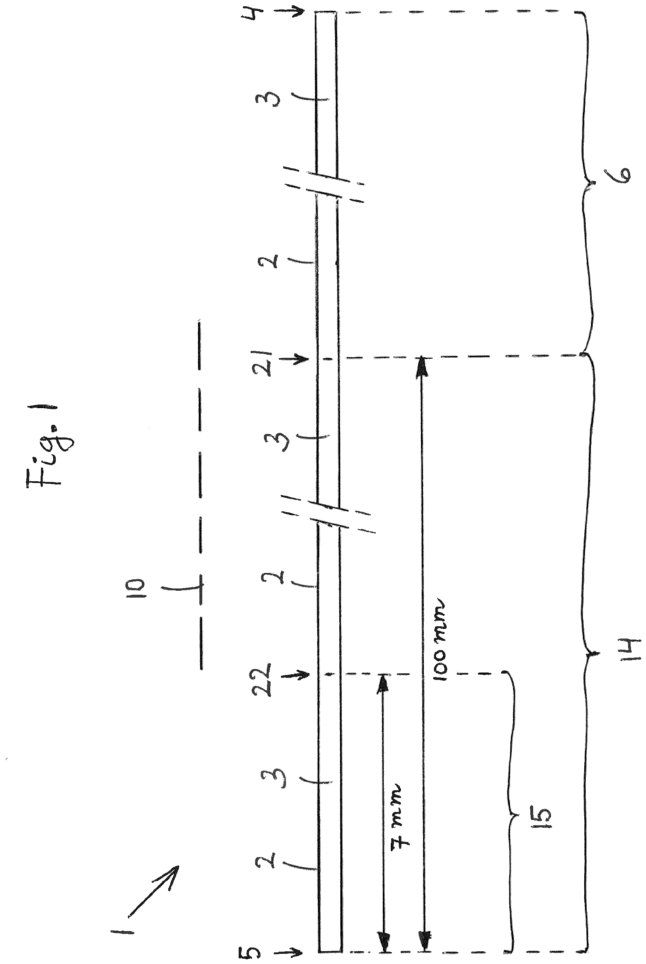

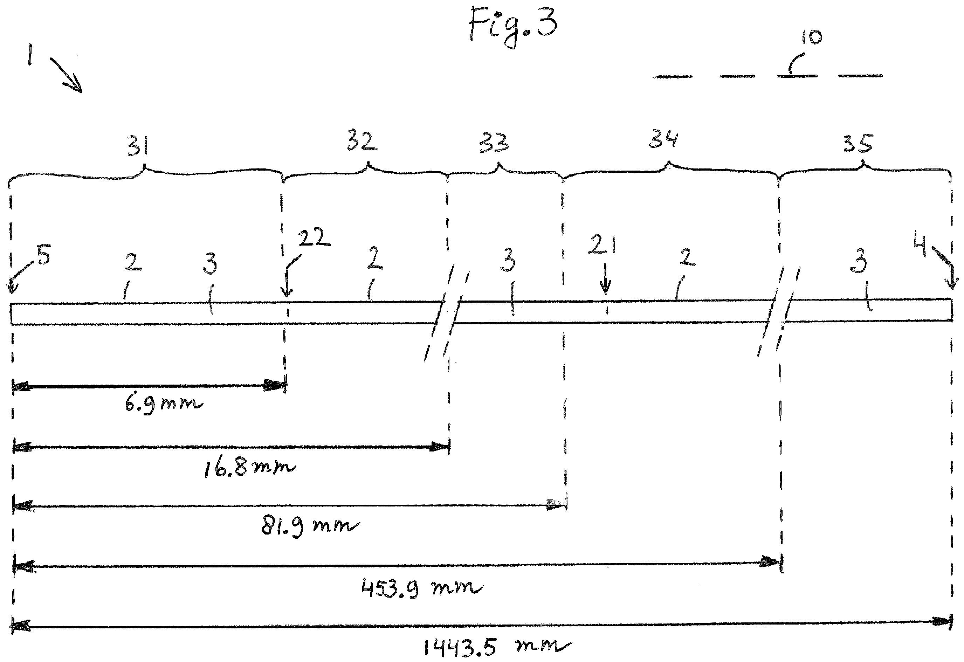

Fig. 3 shows the embodiment of the catheter of Fig. 1 again, and in the same longitudinal side view of Fig. 1, however, wherein this time first, second, third, fourth and fifth longitudinal sub-sections of the catheter wall of the catheter have been indicated by brace marks, wherein the first, second, third, fourth and fifth sub-sections of the catheter wall have first, second, third, fourth and fifth skeleton structures, respectively, of the skeleton of the catheter wall, and wherein said first, second, third, fourth and fifth skeleton structures have mutually different structure types, respectively
Figs. 4A-4C show details of the first skeleton structure of the first sub-section of the catheter wall of the catheter of Fig. 3.
Figs. 5A-5D show details of the second skeleton structure of the second sub-section of the catheter wall of the catheter of Fig. 3.
Figs. 6A-6B show details of the third skeleton structure of the third sub-section of the catheter wall of the catheter of Fig. 3.
Figs. 7A-7E show details of the fourth skeleton structure of the fourth sub-section of the catheter wall of the catheter of Fig. 3.
Figs. 8A-8C show details of the fifth skeleton structure of the fifth sub-section of the catheter wall of the catheter of Fig. 3.

The reference signs used in Figs. 1-8 are referring to the above-mentioned parts and aspects of the invention, as well as to related parts and aspects, in the following manner.
- 1: catheter
- 2: tubular catheter wall
- 3: lumen structure
- 4: proximal end
- 5: distal end
- 6: proximal section
- 7: skeleton
- 7A: cut pattern
- 8: inner liner
- 9: outer laminate
- 10: longitudinal direction
- 11: circumferential direction
- 12: center line
- 14: distal section
- 15: distal tip
- 16: distal skeleton end
- 21: first position
- 22: second position
- 31: first sub-section of catheter wall 2
- 32: second sub-section of catheter wall 2
- 33: third sub-section of catheter wall 2
- 34: fourth sub-section of catheter wall 2
- 35: fifth sub-section of catheter wall 2
- 41: first skeleton structure
- 42: second skeleton structure
- 43: third skeleton structure
- 44: fourth skeleton structure
- 45: fifth skeleton structure
- 51-54: helical parts
- 61-66: helical slots

Based on the above introductory description, including the brief description of the drawing figures, and based on the above-listed reference signs used in Figs. 1-8, the embodiment of Figs. 1-8 is for the greatest part readily selfexplanatory. The following extra explanations are given.

Fig. 1 shows the proximal section 6 and the distal section 14 of the catheter 1 according to the invention. Fig. 1 further shows that the distal section 14 comprises the distal tip 15.

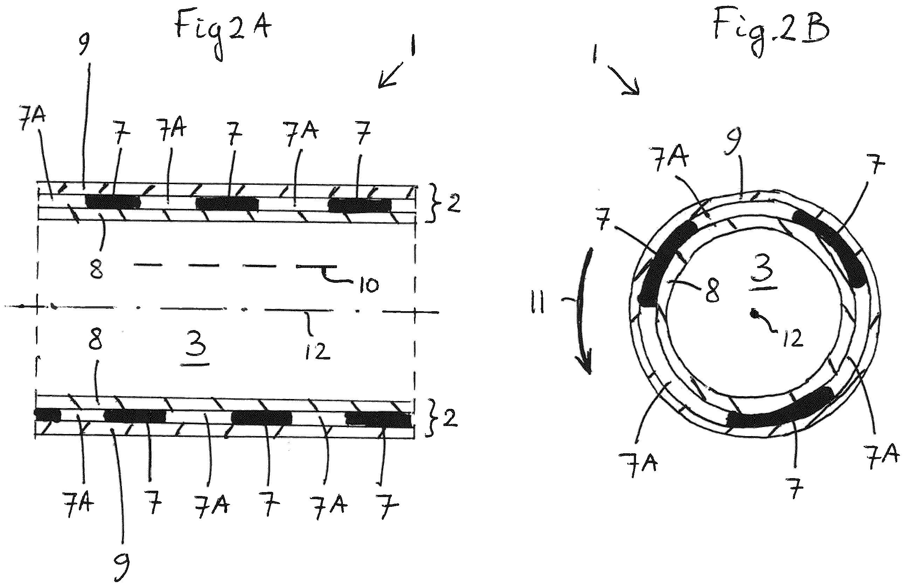

In Figs. 2A-2B it is seen that the catheter wall 2 of the catheter 1 of Fig. 1 has the skeleton 7 comprising the cut pattern 7A. It is further seen that the catheter wall 2 has the inner liner 8 and the outer laminate 9. In the example of Figs. 2A-2B, the lumen structure 3 of the catheter 1 is a single lumen structure. Alternatively, a catheter according to the invention may have a multiple lumen structure.

In the shown example of Figs. 1-8 the skeleton 7, the inner liner 8 and the outer laminate 9 of the catheter wall 2 of the catheter 1 are present along the longitudinal direction 10 in the full range from the proximal end 4 to the distal end 5 of the catheter wall 2. Furthermore, in the shown example of Figs. 1-8, the above-mentioned continuous-skeleton-range in which the skeleton is continuously extending to form at least one elongated continuous reinforcement structure all along said continuous-skeleton-range, is extending in the full range from the proximal end 4 to the distal end 5 of the catheter wall 2. Alternatively, however, for a catheter according to the invention the continuous-skeleton-range does not necessarily have to extend in the full range from the proximal end to the distal end of the catheter wall of the catheter, and also the skeleton, the inner liner and the outer laminate of a catheter according to the invention do not necessarily have to be present in the full range from the proximal end to the distal end of the catheter wall, as long as the skeleton, the inner liner and the outer laminate are at least present in a continuous-skeleton-range in a manner as specified according to the invention in the above introduction of the present disclosure. So, for a catheter according to the invention the continuous-skeleton-range could, for example, be confined to extending distally along the longitudinal direction from the first position 21 to a position at 2 mm proximal from the distal end.

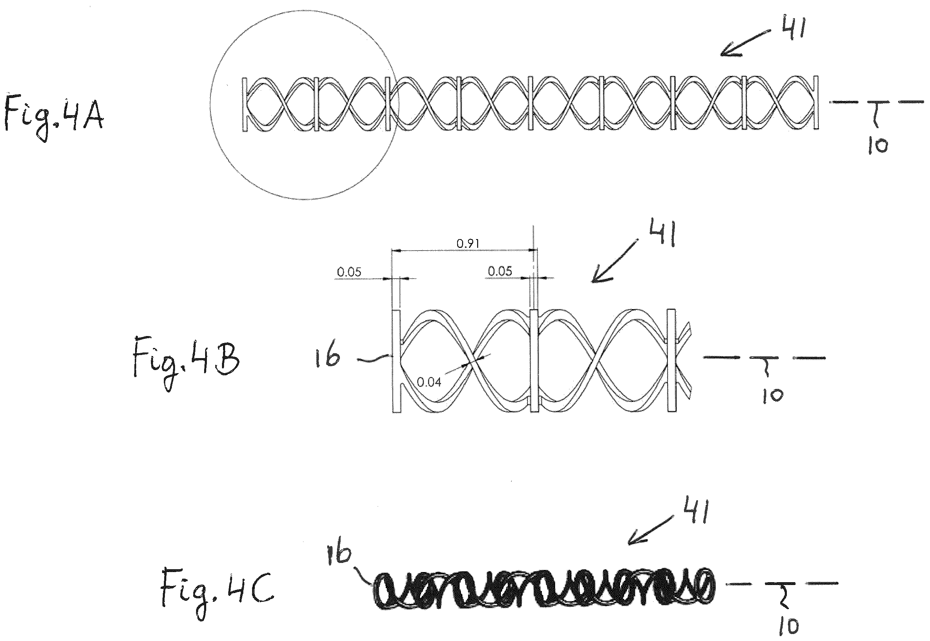

Fig. 3 shows the first sub-section 31, the second sub-section 32, the third sub-section 33, the fourth sub-section 34 and the fifth sub-section 35 of the catheter wall 2, having the first skeleton structure 41, the second skeleton structure 42, the third skeleton structure 43, the fourth skeleton structure 44 and the fifth skeleton structure 45, respectively, which are shown in more detail in Figs. 4-8, respectively.

The five skeleton structures 41-45 are successively interconnected in an end-to-end manner along the longitudinal direction 10 to form a string that forms the skeleton 7 of the catheter wall 2 of the catheter 1.

It is noted that, in the shown example, the outer diameter and the inner diameter of the catheter wall 2 are 0.635 mm and 0.510 mm, respectively, in the full range along the longitudinal direction 10 from the proximal end 4 to the distal end 5 of the catheter wall 2. Alternatively, however, for a catheter according to the invention, neither the outer diameter nor the inner diameter of the catheter wall 2 has to be constant in the full range from the proximal end to the distal end of the catheter wall. For example, as mentioned above, the distal tip 15 may be a tapered distal tip in that the distal tip has an outer diameter, which, distally along the longitudinal direction, is gradually narrowing up to the distal end 5.

Furthermore it is noted that, if the distal tip 15 is a tapered distal tip, and if a radiopaque element is embedded in the catheter wall in that the radiopaque element is located in a space of the cut pattern 7A, the embodiment of Figs. 1-8 is an embodiment of a catheter according to all of the preferable embodiments of the present invention, which are mentioned in the above introduction of the present disclosure.

Reference is now made to Figs. 4A-4C, which show the first skeleton structure 41 of the skeleton 7. Fig. 4A is a longitudinal side view of the first skeleton structure 41 along the full range of extent of the first sub-section 31 along the longitudinal direction 10, which corresponds to a length of 6.9 mm as indicated in Fig. 3. Fig. 4B is an enlarged detail of the encircled area on the left side of Fig. 4A. In Fig. 4B some dimensions have been indicated by numerical values, which are to be interpreted in millimeters (mm). Fig. 4C is a perspective view of the first skeleton structure 41. In Figs. 4A-4C the first skeleton structure 41 consists of nine parallel rings which in the longitudinal direction are transversely inter-connected by pairs of helical parts. Figs. 4A-4C further show the distal skeleton end 16 in the form of a circular edge of an end ring of said nine rings of the first skeleton structure 41. This distal skeleton end 16 in the form of a circular edge of the skeleton 7 promotes good penetration behaviour of the distal tip 15 to successfully pass highly clogged stenoses, while at the same time improving the structural integrity of the skeleton 7, and while still allowing for good flexibility of the distal tip 15.

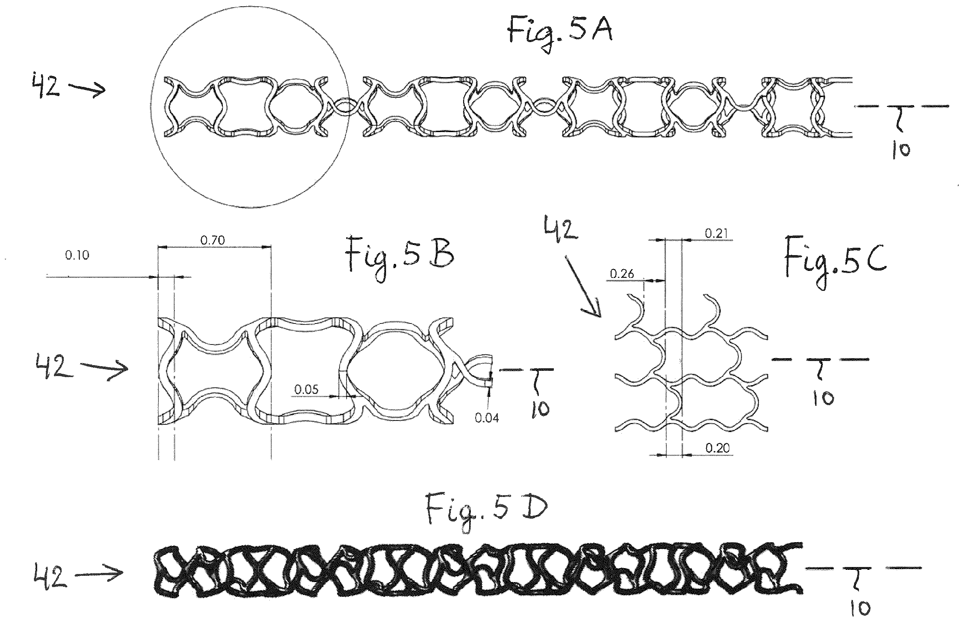

Reference is now made to Figs. 5A-5D, which illustrate the second skeleton structure 42 of the skeleton 7. The second skeleton structure 42 is extending in the second sub-section 32 along the longitudinal direction 10 over a length of 16.8 - 6.9 mm = 9.9 mm, as indicated in Fig. 3. Each of Figs. 5A-5D shows the second skeleton structure 42 only along a part of the last-mentioned length. Fig. 5A shows a part of the second skeleton structure 42 in longitudinal side view. Fig. 5B is an enlarged detail of the encircled area on the left side of Fig. 5A. Fig. 5C shows a top view on a longitudinal section of the second skeleton structure 42 in an imaginary "unrolled tube" state of the longitudinal section. To explain the term "unrolled tube" it is noted that the skeleton according to the invention is a tubular wall (more particularly an interrupted tubular reinforcement wall), and that such a tubular wall imaginarily can be unrolled into a completely straight state by imaginarily unrolling it onto a fully straight plane after an imaginary straight cut, parallel to the center line, has been made over the full length of the tubular wall. In Figs. 5B and 5C some dimensions have been indicated by numerical values, which are to be interpreted in millimeters (mm). Fig. 5D is a perspective view of the part of the second skeleton structure 42 shown in Fig. 5A.

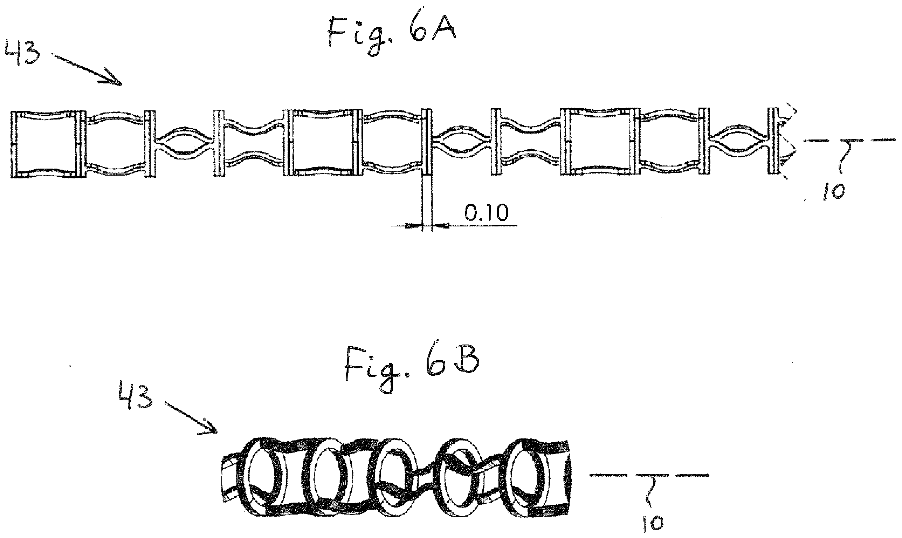

Reference is now made to Figs. 6A-6B, which illustrate the third skeleton structure 43 of the skeleton 7. The third skeleton structure 43 is extending in the third sub-section 33 along the longitudinal direction 10 over a length of 81.9 - 16.8 mm = 65.1 mm, as indicated in Fig. 3. Each of Figs. 6A-6B shows the third skeleton structure 43 only along a part of the last-mentioned length. Fig. 6A shows a part of the third skeleton structure 43 in longitudinal side view. Fig. 6B is a perspective view of a part of the third skeleton structure 43. Figs. 6A-6B show that the third skeleton structure 43 of the skeleton 7 consists of parallel rings which in axial direction are transversely inter-connected by pairs of diametrically opposite springlike connection beams, wherein successive beam pairs in the longitudinal direction each time are positioned with 45 degrees offset angle relative to one another in circumferential direction around the longitudinal direction. In Fig. 6A a thickness of one of the parallel rings has been indicated by the numerical value 0.10, which is to be interpreted in millimeters (mm).

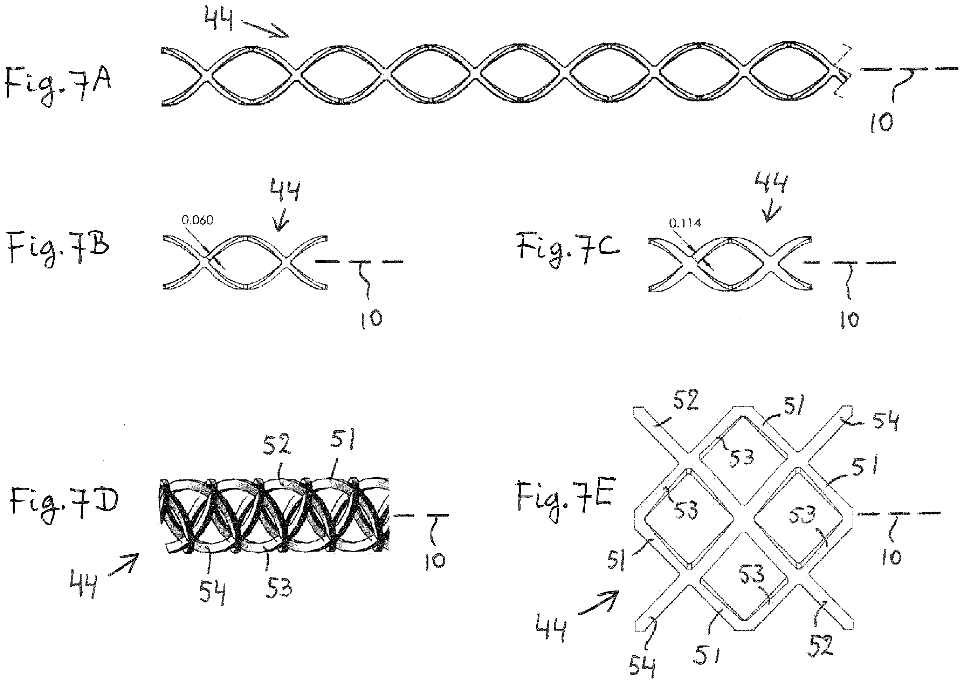

Reference is now made to Figs. 7A-7E, which illustrate the fourth skeleton structure 44 of the skeleton 7. The fourth skeleton structure 44 is extending in the fourth sub-section 34 along the longitudinal direction 10 over a length of 453.9 - 81.9 mm = 372.0 mm, as indicated in Fig. 3. Each of Figs. 7A-7E shows the fourth skeleton structure 44 only along a part of the last-mentioned length. Fig. 7A shows a part of the fourth skeleton structure 44 in longitudinal side view. Figs. 7B and 7C show two other parts of the fourth skeleton structure 44 in longitudinal side view, wherein the part of Fig. 7B is located distally from the part of Fig. 7A, and the part of Fig. 7C is located proximally from the part of Fig. 7A. Fig. 7D is a perspective view of the fourth skeleton structure 44. Fig. 7E shows a top view on a longitudinal section of the fourth skeleton structure 44 in an imaginary unrolled tube state of the longitudinal section (the term "unrolled tube" is explained above with reference to Fig. 5C). Figs. 7D-7E show that the fourth skeleton structure 44 of the skeleton 7 consists of four helical parts, two of which being right-handed helical parts 51, 52 and two of which being left-handed helical parts 53, 54, wherein the two right-handed helical parts 51, 52 are intersecting the two left-handed helical parts 53, 54 at multiple nodes of the fourth skeleton structure 44.

In Figs. 7B and 7C some dimensions have been indicated by numerical values, which are to be interpreted in millimeters (mm). Figs. 7B and 7C serve to illustrate the aspect that the thickness of the helical parts of the fourth skeleton structure 44 gradually decreases distally along the longitudinal direction. Thereby, the flexibility of the fourth sub-section 34 of the catheter wall 2 gradually increases distally along the longitudinal direction 10.

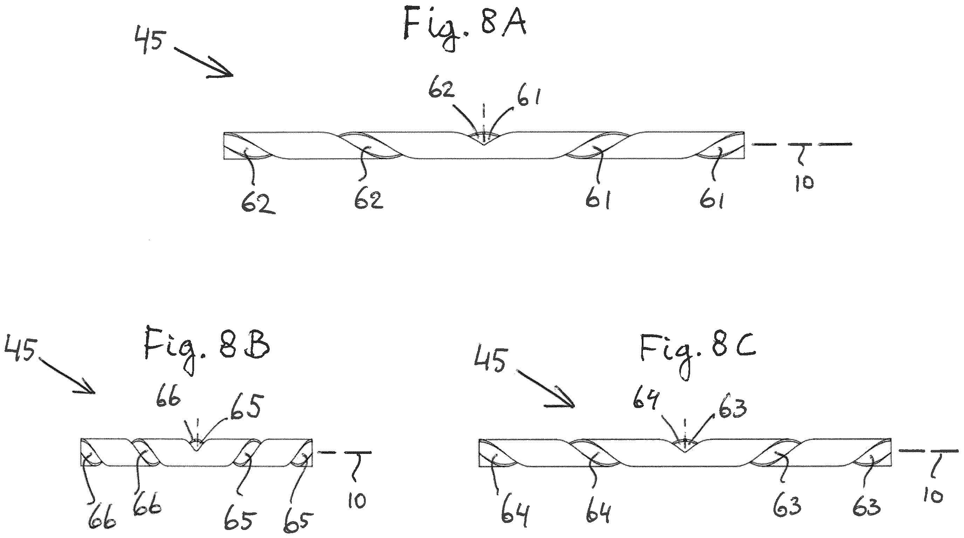

Reference is now made to Figs. 8A-8C, which show the fifth skeleton structure 45 of the skeleton 7 in longitudinal side view. The fifth skeleton structure 45 is extending in the fifth sub-section 35 along the longitudinal direction 10 over a length of 1443.5 - 453.9 mm = 989.6 mm, as indicated in Fig. 3. Each of Figs. 8A-8C shows the fifth skeleton structure 45 only along a part of the last-mentioned length. The part of Fig 8C is located distally far away from the part of Fig. 8A. The part of Fig. 8B is located distally far away from the part of Fig. 8C. The fifth skeleton structure 45 of the skeleton 7 has a number of helical slots which are interconnected in an end-to-end manner along the longitudinal direction 10. Each helical slot has only a few helical windings. For example, Fig. 8A shows the helical slots 61 and 62, Fig. 8C shows the helical slots 63 and 64, and Fig. 8B shows the helical slots 65 and 66. Two mutually connected ones of the helical slots each time have opposite winding directions relative to one another. Such connections of two mutually helical slots are shown in the longitudinal middle of each of the Figs. 8A-8C. Figs. 8A-8C further serve to illustrate that the pitch of the helical slots gradually decreases for distally subsequent helical slots along the longitudinal direction 10. Due to said decreasing pitch, the overall skeleton cut-away ratio of the fifth sub-section 35 of the catheter wall 2 gradually increases distally, with the effect that the flexibility of the fifth sub-section 35 of the catheter wall 2 gradually increases distally along the longitudinal direction.

Figs. 1-8 illustrate that the skeleton of a catheter according to the invention may at least partly be formed by many various skeleton (sub)structures, such as the shown skeleton structures 41-45 of Figs. 4-8, respectively.

The invention can be practiced with many various structures, many various materials, many various shapes and many various dimensions of the skeleton, inner liner and outer laminate of the catheter, and with many various additional features and many various accessories of the catheter, such as the many various structures, the many various materials, the many various shapes and the many various dimensions of skeletons, inner liners and outer laminates, and the many various additional features and the many various accessories that are used in known microcatheters for interventional cardiovascular and/or neurovascular applications, as long as the catheter is within the scope of the appended claims.

It is noted that the first position at 100 mm proximal from the distal end, i.e. at the transition between the proximal section and the distal section, does not necessarily imply a transition in building structure between the proximal section and the distal section. Similarly, it is noted that the second position at 7 mm proximal from the distal end, i.e. at the transition between the distal tip and the remainder of the distal section, does not necessarily imply a transition in building structure between the distal tip and the remainder of the distal section. In fact, as used herein, the definitions of the proximal section, the distal section and the distal tip, merely serve to quantify the overall skeleton cut-away ratios of the distal section and the distal tip, respectively, of a catheter according to the invention.

Regarding the overall skeleton cut-away ratio the following general remark is made. If, everywhere in a longitudinally continuous longitudinal section of a catheter wall, the catheter wall has a totally uninterrupted tubular metal reinforcement wall, for example in the form of a totally uninterrupted metal hypotube, then said longitudinal section has an overall skeleton cut-away ratio of 0%.

It is further noted that according to the invention typical dimensions of some parts and aspects of the invention, and applicable practical ranges of such dimensions are as follows. Effective length of the catheter (as measured from the distal end of a hub of the catheter) can be in the range between 130 cm and 160 cm, and can typically be 135 cm. Maximum outer diameter of the first distal section of the tubular catheter wall of the catheter can be in the range between 0.60 mm and 1.20 mm, and can typically be 0.75 mm, while noting that the word "maximum" in the term "maximum outer diameter" is referring to the maximum value as considered over the full length of the first distal section. Minimum inner diameter of the first distal section of the tubular catheter wall of the catheter can be in the range between 0.36 mm and 0.55 mm, and can typically be 0.45 mm, while noting that the word "minimum" in the term "minimum inner diameter" is referring to the minimum value as considered over the full length of the first distal section. For example, the following typical combinations of maximum outer diameter and minimum inner diameter of the first distal section are possible: maximum outer diameter 0.75 mm in combination with minimum inner diameter 0.45 mm, maximum outer diameter 1.20 mm in combination with minimum inner diameter 0.55 mm, or maximum outer diameter 0.60 mm in combination with minimum inner diameter 0.36 mm.

## Claims

1. A catheter (1), being a microcatheter for interventional cardiovascular and/or interventional neurovascular applications,
wherein the catheter comprises a tubular catheter wall (2), which is surrounding a lumen structure (3) of the catheter, the catheter wall having a proximal end (4), a distal end (5) and a longitudinal direction (10) extending from the proximal end to the distal end, wherein the catheter wall has a length of at least 1000 mm from the proximal end (4) to the distal end (5) along the longitudinal direction;
wherein the catheter wall (2) comprises:
- a proximal section (6), being defined as extending distally along the longitudinal direction from the proximal end (4) to a first position (21) at 100 mm proximal from the distal end (5), and
- a distal section (14), being defined as extending distally along the longitudinal direction from said first position (21) to the distal end (5),
wherein the distal section (14) comprises a distal tip (15) of the catheter (1), which is defined as extending proximally along the longitudinal direction (10) from the distal end (5) to a second position (22) at 7 mm proximal from the distal end (5);
and wherein the catheter wall (2) at least in a part of the distal section (14) comprises:
- a skeleton (7), which provides reinforcement of the catheter wall (2) in the longitudinal direction (10), as well in a circumferential direction (11) around the longitudinal direction (10), wherein the skeleton (7) is an interrupted tubular reinforcement wall obtained by microfabricating a cut pattern (7A) in a fully uninterrupted tubular reinforcement wall made of a reinforcement material, and wherein the reinforcement material of the skeleton, everywhere in a continuous-skeleton-range along the longitudinal direction, is continuously extending to form at least one elongated continuous reinforcement structure all along said continuous-skeleton-range, wherein said continuous-skeleton-range is at least extending distally along the longitudinal direction from the first position (21) to a position at 2 mm proximal from the distal end (5),
- an inner liner (8), which at least in said continuous-skeleton-range is co-axially surrounded by said skeleton (7) relative to a center line (12) of the catheter wall (2), and
- an outer laminate (9), which at least in said continuous-skeleton-range is co-axially surrounding said skeleton (7) relative to the center line (12) of the catheter wall;
and wherein an overall skeleton cut-away ratio of a longitudinal section of the catheter wall (2) in a longitudinal range along the longitudinal direction (10) is defined as the percentage of:
- the volume of all parts, within said longitudinal range, of said reinforcement material being cut-away from said fully uninterrupted tubular reinforcement wall according to said microfabricated cut pattern (7A), relative to
- the volume of all parts, within said longitudinal range, of said reinforcement material of said fully uninterrupted tubular reinforcement wall;
and wherein the overall skeleton cut-away ratio of all parts of the distal section (14) that are within said continuous-skeleton-range is at least 50%,
and wherein the overall skeleton cut-away ratio of all parts of the distal tip (15) that are within said continuous-skeleton-range is at least 20%.

2. The catheter (1) according to claim 1, wherein said continuous-skeleton-range is at least extending distally along the longitudinal direction (10) from the first position (21) to a position at 0.5 mm proximal from the distal end (5).

3. The catheter (1) according to claim 2, wherein said continuous-skeleton-range is at least extending distally along the longitudinal direction (10) from the first position (21) to a position at 0.2 mm proximal from the distal end (5).

4. The catheter (1) according to any one of the preceding claims,
wherein the overall skeleton cut-away ratio of said all parts of the distal section (14) that are within said continuous-skeleton-range is at least 65%, and
wherein the overall skeleton cut-away ratio of said all parts of the distal tip (15) that are within said continuous-skeleton-range is at least 40%.

5. The catheter (1) according to claim 4,
wherein the overall skeleton cut-away ratio of said all parts of the distal section (14) that are within said continuous-skeleton-range is at least 80%, and
wherein the overall skeleton cut-away ratio of said all parts of the distal tip (15) that are within said continuous-skeleton-range is at least 60%.

6. The catheter (1) according to any one of the preceding claims, wherein a radiopaque element is embedded in the catheter wall (2) in that the radiopaque element is located in a space of the cut pattern (7A), said space being bounded by the skeleton (7), the inner layer (8) and the outer laminate (9).

7. The catheter (1) according to any one of the preceding claims, wherein the skeleton (7), distally along the longitudinal direction (10), has a distal skeleton end (16) in the form of a circular edge of the skeleton, said circular edge having a center point at the center line (12) of the catheter wall (2).

8. The catheter (1) according to any one of the preceding claims, wherein the distal tip (15) is a tapered distal tip in that the distal tip (15) has an outer diameter, which, distally along the longitudinal direction (10), is gradually narrowing up to the distal end (5).
